# NOUVEAU FASCICULE DE BREVET EUROPEEN

(11) **EP 0 480 982 B2**
(45) Date de publication et mention de la décision concernant l'opposition: **12.11.1997**
(45) Mention de la délivrance du brevet: 20.10.1993
(21) Numéro de dépôt: 90910773.2
(22) Date de dépôt: 29.06.1990
(51) Int. Cl.: A61K 39/39, A61K 9/107

(54) **VACCINS ET VECTEURS DE PRINCIPES ACTIFS FLUIDES CONTENANT UNE HUILE METABOLISABLE**
EIN METABOLISIERBARES ÖL ENTHALTENDE IMPFSTOFFE UND WIRKSTOFFTRÄGER
VACCINES AND VECTORS WITH LIQUID ACTIVE PRINCIPLES CONTAINING AN OIL WHICH CAN BE METABOLISED

(30) Priorité: 03.07.1989 FR 8908918
(43) Date de publication de la demande: 22.04.1992
(73) Titulaire: S.E.P.P.I.C., SOCIETE D'EXPLOITATION DE PRODUITS POUR LES INDUSTRIES CHIMIQUES, F-75321 Paris Cédex 07 (FR)
(72) Inventeur: BRANCQ, Bernard, F-78150 Le Chesnay (FR); TROUVE, Gérard, F-81100 Castres (FR)
(74) Mandataire: Portal, Gérard
(86) Numéro de dépôt international: FR9000485
(87) Numéro de publication internationale: WO9100107

(56) Documents cités:
- FR-A- 2 342 073
- FR-A- 2 501 526
- US-A- 3 678 149
- US-A- 3 983 228
- US-A- 4 803 070
- J. Vet. Med. B33, 1986, pp. 340-345
- Laboratory Animal Science, vol. 39, no 3, mai 1989, pp. 222-225
- Merck Index, 11ème éd., 5231-Lanolin et 8727-Squalene

## Description

La présente invention concerne de nouvelles compositions de vaccins et d'injectables fluides, contenant une phase huileuse totalement ou partiellement métabolisable, qui peuvent être utilisés en médecine vétérinaire ou humaine.

La vaccination est un moyen de lutte préventive contre les infections visant à mettre en éveil le systeme de défense immunitaire, par injection d'antigènes, qui sont souvent des fractions très spécifiques des parois virales ou bactériennes.

Les progrès du génie génétique font que les antigènes disponibles sont de plus en plus purifiés, voire totalement synthétiques. Cette pureté est un atout incontestable sur le plan de la sécurité du vaccin, mais elle s'accompagne en général d'une diminution de l'efficacité immunologique.

Aussi, la demande d'adjuvants d'immunité, permettant d'accroître les réponses du système immunitaire à un antigène, est-elle de plus en plus forte. Cependant, la présence de ces adjuvants ne doit pas aller à l'encontre de la bonne innocuité des préparations antigéniques modernes.

De très nombreux adjuvants d'immunité ont été décrits; mais seuls quelques-uns d'entre eux sont utilisés au stade des vaccins industriels, et seuls l'hydroxyde et le phosphate d'aluminium sont autorisés en médecine humaine.

Il est cependant reconnu que les vaccins "huileux", dans lesquels le milieu antigénique est émulsionné avec une huile minérale contenant un émulgateur, sont les plus efficaces, notamment Lorsque ces vaccins sont de type E/H.

L'adjuvant résultant de l'association d'huile minérale et d'un ester du mannitol est connu sous l'appellation d'adjuvant incomplet de Freund (FIA : "Freund's incomplete adjuvant" dans la Littérature) ; sa composition est :
- - huile minérale fluide (type Bayol F) :: 85 %
- - (Arlacel A) monooléate de mannide :: 15 %

Par distinction, on nomme FCA, l'adjuvant de Freund "complet" qui contient, outre les éléments précités, une mycobactérie tuberculineuse qui potentialise l'effet immunitaire.

Ces adjuvants FIA et FCA, connus de longue date, restent aujourd'hui les produits de référence dans le monde entier pour des études immunologiques de Laboratoire.

On les trouve en particulier associés à des adjuvants injectables originaux, tels que les muramyl dipeptides (MDP) ou des antigènes obtenus par synthèse chimique ou génétique (VP1, etc.).

L'utilisation industrielle des vaccins contenant FIA et FCA est toutefois limitée en raison de la difficulté de leur mise en oeuvre dans des préparations vaccinales injectables et de leur mauvaise tolérance par les sujets vaccinés.

En effet, il est connu que les émulsions huileuses obtenues à partir de FIA et FCA sont visqueuses comme de la mayonnaise et relarguent de l'huile. En conséquence, ces préparations sont difficilement injectables à travers des seringues de faible diamètre d'aiguille (0,2 mm).

De plus, elles créent, au niveau du site d'injection chez les animaux, des réactions locales avec oedèmes et abcès non acceptables par les Autorités Sanitaires qui rendent l'animal impropre à la consommation en boucherie.

Des préparations vaccinales antidiphtériques réalisées avec FCA injectées à des femmes ont même provoqué des intolérances graves, des syndromes d'avortement qui ont prohibé ces adjuvants pour une utilisation courante chez l'être humain.

L'exemple d'un vaccin contre la fièvre aphteuse contenant du FIA est décrit ci-dessous afin de mettre en évidence l'efficacité immunologique, les propriétés physico-chimiques et la mauvaise tolérance de ce type de préparation.

### Exemple 1

On réalise une préparation vaccinale pour le traitement de la fièvre aphteuse chez les bovins à partir d'adjuvant de Freund incomplet (FIA) par mélange sous agitation mécanique d'une partie de FIA et d'une partie de milieu antigénique inactivé.

La préparation du milieu antigénique a fait l'objet d'un soin particulier lors de la culture, de la purification et de la concentration des antigènes qui sont dilués dans un milieu type Eagle en présence de tampon phosphaté.

La composition de la préparation est donc (poids/poids) :
- Milieu antigénique: 50 %
- Huile minérale: 42,5 %
- Monooléate de mannide: 7,5 %

Les caractéristiques physico-chimiques obtenues sur cette préparation sont :
- Conductivité à 20°C :: 12 microsiemens
- Viscosité à 20°C :: 6 500 centistokes
- pH:: 7,6
- Stabilité :: relargage d'huile au stockage à 4°C
- Aspect microscopique :: gouttelettes de taille supérieure à 2 µm

Les tests d'efficacité immunologique chez le bovin montrent une bonne réponse immunitaire avec production persistante d'anticorps circulant 30 jours après la vaccination intramusculaire.

### Expérimentation :

Résultats immunitaires fièvre aphteuse bovins
- vaccins huileux adjuvant FIA- Dose injectée 5 ml
- les résultats (EPP) sont exprimés en pourcentage de nombre d'animaux protégés vis-à-vis des virus de valences 0, A et C, sur une durée de 3 mois, avec des vaccins de plusieurs lots, stockés à 4°C.

**TABLEAU 1**

| EPP* | | Vaccin n° 1 stocké 1 mois à 4°C | Vaccin n° 2 stocké 24 mois à 4°C |
|---|---|---|---|
| Virus 0 | 30 jours * | 79 | 77 |
| | 90 jours | 60 | 76 |
| A | 30 jours | 85 | 80 |
| | 90 jours | 70 | 69 |
| C | 30 jours | 93 | 95 |
| | 90 jours | 72 | 88 |

| | | | |
|---|---|---|---|
| * mesure de la protection 30 et 90 jours après vaccination pour mesurer l'effet prolongé de l'immunité. | | | |

L'examen microscopique des effets toxiques sur l'animal de la préparation vaccinale au point d'injection monde la présence importante de nodules et de granulomes et même d'abcès ouverts. L'étendu des régions lesées est importante.

Un examen histologique met en évidence la présence de résidus huileux dans les tissus et une notable augmentation de la taille des ganglions externes.

Pour améliorer l'injectabilité de tels vaccins huileux, une technique connue consiste à incorporer, dans le milieu antigénique, une faible proportion d'un émulgateur hydrophile, le Polysorbate 80 (décrit dans les pharmacopées).

La présence de ce produit réduit de façon importante la viscosité de vaccins comme il est montré dans l'exemple 2.

Toutefois, on sait que le polysorbate 80, utilisé en biochimie comme agent délipidant, est agressif vis-à-vis des parois cellulaires et donc potentiellement toxique.

### Exemple 2

La préparation vaccinale réalisée dans l'exemple 1 a été modifiée en ajoutant 1 % de Polysorbate 80 à l'adjuvant FIA.

La composition de la préparation devient :
- Milieu antigénique :: 49,5 %
- Huile minérale :: 42,5 %
- Monooléate de mannide :: 7,5%
- Polysorbate 80 :: 0,5 %

Les caractéristiques physico-chimiques obtenues sont :
- Conductivité à 20°C :: 10 microsiemens
- Viscosité à 20°C :: 350 centistokes
- pH:: 7,6
- Stabilité :: léger anneau d'huile au stockage à 4°C

La mesure de la réponse immunitaire à l'injection intramusculaire chez le bovin est sensiblement équivalente à celle obtenue avec la préparation de l'exemple 1, lorsque l'on utilise une préparation selon l'exemple 2, réalisée depuis moins de 1 mois.

Par contre, lorsque l'on utilise une préparation selon l'exemple 2 réalisée depuis 1 an et stockée à 4°C dans des conditions stériles, on constate une altération de la phase antigénique et une diminution de la réponse immunitaire avec un taux d'anticorps circulants moins élevé.

Ces résultats sont expliqués par la présence de l'émulsionnant hydrophile Polysorbate 80 (caractérisé par HLB de 15) qui exerce un pouvoir mouillant sur les enveloppes cellulaires des antigènes et solubilise les proé téines de surface. Ceci a pour effet de les dénaturer et de modifier leur aptitude à la création d'anticorps.

Ce mécanisme des agents tensioactifs hydrophiles vis-à-vis des micro-organismes est connu et décrit en particulier dans la littérature.

Au niveau des résultats de toxicité à l'injection chez l'animal, on observe des phénomènes similaires à ceux obtenus avec la préparation de l'exemple 1, qui sont caractéristiques d'une mauvaise tolérance du vaccin.

On trouve des préparations vaccinales commercialisées selon le procédé de l'exemple 2, associant de l'adjuvant FIA et un émulsionnant hydrophile. Le brevet belge BE-A-648053 de Philips décrit un vaccin contre la brucellose réalisé dans des conditions similaires, et qui présente une notable réduction de la viscosité par rapport aux préparations réalisées avec l'adjuvant FIA seul.

L'utilisation d'huiles métabolisables telles qu'huiles végétales, squalène, squalane à la place d'huiles minérales a permis dans certains cas d'obtenir des vaccins de type E/H bien mieux tolérés. Weilbel et coll. ne notent que de petits nodules de 3 à 4 mm chez les humains vaccinés au moyen d'un vaccin grippal contenant de l'huile d'arachide (P.S.E.B.H 143, 1053 - 1056 (1973), cependant, les réponses immunitaires enregistrées sont en général plus faibles et la protection moins longue avec ce type de vaccins qu'avec les vaccins sur huile minérale. Ceci peut être expliqué par une dégradation plus rapide du vaccin dans l'organisme.

Les exemples rapportés dans le brevet anglais 1 081 796 (1965) de L.B HOLT sont bien représentatifs de ce phénomène (tableau A)

**Tableau A**

| Evolution du taux d'anticorps avec 2 vaccins huileux adjuvés FIA ou huile d'amande douce. TAUX D'ANTICORPS | | | | | | |
|---|---|---|---|---|---|---|
| ANTIGENE | DIPHTERIA TOXOID | | TETANUS TOXOID | | B PERTUSIS | |
| | 6 sem. | 13 sem. | 6 sem. | 13 sem. | 6 sem. | 13 sem. |
| ADJUVANT | | | | | | |
| FIA | 0,32 | 0,26 | 5,0 | 0,41 | 20 | 431 |
| huile végétale + mannide mono-oléate | 0,10 | 0,07 | 0,30 | 0,20 | 20 | 31 |

Une autre caractéristique particulièrement désavantageuse de ces vaccins sur huile métabolisable est leur viscosité qui rend leur injection difficile et douloureuse. Les vaccins adjuvés par du squalène ou du squalane décrit dans le brevet européen 0 117 934 ont une viscosité supérieure à 4000 mPa.s.

De même, ceux décrits dans l'article de WOODHOUR (PSEBM 116, 516-523, 1964) et contenant de l'huile d'arachide et du monostéarate d'aluminium sont présentés comme "highly viscous WATER-in-oil émulsion".

Il a été trouvé, de façon tout à fait inattendue, que l'adjonction dans certaines conditions d'une huile métabolisable aux vaccins contenant une huile minérale (ou de manière générale non métabolisable) permettait d'en améliorer considérablement la tolérance sans en modifier de façon significative l'efficace immunologique à condition que les vaccins obtenus soient stables et très fluides.

L'obtention de ces préparations fluides, stables, totalement ou partiellement métabolisables, a été rendue possible grâce à l'utilisation d'émulgateurs non toxiques, faiblement hydrophiles, obtenus à partird'acides gras liquides, de mannitol ou de glycérol. L'objet de l'invention est décrit dans les revendications.

Les vaccins (ou préparations injectables) de l'invention sont caractérisés par 4 grandeurs physico-chimiques importantes :
- fluidité : la viscosité mesurée par un appareil à mobile tournant du type BROOKFIELD doit être inférieure à 800 centistokes à 20°C pour être injectable à travers une aiguille de seringue dont le diamètre est de 0,2 mm;
- stabilité : la préparation émulsionnée ne doit pas déphaser, c'est-à-dire libérer la phase antigénique interne ni la phase huileuse dans des conditions de stockage normales pour ce type de produit;
- conductivité : elle détermine le caractère huileux ou aqueux de la phase continue de l'émulsion. Pour des valeurs inférieures à 20 microsiemens environ, on obtient une phase continue huileuse à température ambiante;
- aspect microscopique : les tailles de gouttelettes de phase antigénique dispersées sont inférieures à 10 µm et peu dispersées.

Mis à part le milieu antigénique, ou les principes actifs spécifiques à chaque préparation injectable, les autres constituants (huiles, émulgateurs) doivent répondre à des critères précis définis ci-après.

### Définition de l'huile

Les huiles fluides sont choisies parmi les huiles minérales, végétales ou animales connues pour leur faible toxicité. Elles doivent être liquides à la température de stockage (+4°C) ou, au moins, donner des émulsions liquides à cette température. On choisira en particulier des huiles minérales à chaîne linéaire ayant un nombre d'atomes de carbone supérieur à 16 et exemptes de composés aromatiques.

Des exemples connus sont le MARCOL® 52 (produit par ESSO France) et le DRAKEOL®6VR (produit par PENRECO USA).

On peut également utiliser des huiles minérales synthétiques telles que les polyisobutènes ou les polyisoprènes.

Parmi les huiles végétales, on choisira des huiles insaturées riches en acide oléique qui sont biodégradables et connues pour leur pouvoir immunogéne, par exemple les huiles d'arachide, d'olive, de sésame, de soja, de germe de blé, etc.

Pour les huiles animales, les mêmes critères de tolérance et d'efficacité immunologique permettent d'utiliser par exemple du squalène, du squalane, de l'huile de spermaceti.

Un mélange des huiles citées précédemment est avantageusement utilisé dans le cadre de la présente invention.

### Définition du système émulgateur

Il doit être adapté pour donner des préparations injectables de type E/H fluides et stables.

Il se compose d'un ou plusieurs agents tensioactifs ayant en mélange un caractère lipophile ou faiblement hydrophile caractérisé par un nombre HLB (Hydrophile - Lipophile - Balance) compris entre 2 et 9. Les émulgateurs sont de préférence obtenus par condensation d'un acide gras liquide à 20°C sur un sucre (mannitol, glucose, saccharose) ou du glycérol. Les acides gras préférés sont ceux ayant au moins 16 atomes de carbone et en particulier les acides oléique, linoléique, ricinoléique, cétostéarique.

On utilise de préférence les esters de mannitol notamment les oléates obtenus dans des conditions de synthèses particulières, par anhydrisation de la chaîne carbonée polyhydroxylée du mannitol qui se cyclise en 1-4 ou en 2-6. L'hydrophilie des esters obtenus peut être modifiée par greffage de fonctions hydrophiles telles qu'alcool, polyols, oxyde d'éthylène, oxyde de propylène, acide carboxylique, amine, amide...

Tous les émulgateurs utilisés doivent être pharmaceutiquement acceptables pour un usage en injectable, notamment être dépourvus de métaux lourds, et présenter des indices d'acide ou de peroxyde très faibles. Il est également souhaitable qu'ils satisfassent les normes de tests d'innocuité tels que, par exemple, celui décrit par S.S. BERLIN (annals of allergy 20, 473 1962).

Il est également souhaitable que les émulgateurs utilisés forment avec l'huile choisie une phase homogène, limpide et stable qui sera émulsionnée avec le milieu aqueux propre à chaque préparation injectable.

Les émulgateurs décrits dans le tableau B répondent aux exigences ci-dessus. Ils sont utilisés dans les préparations injectables décrites dans les exemples 3 à 9 pour former des émulsions fluides et stables.

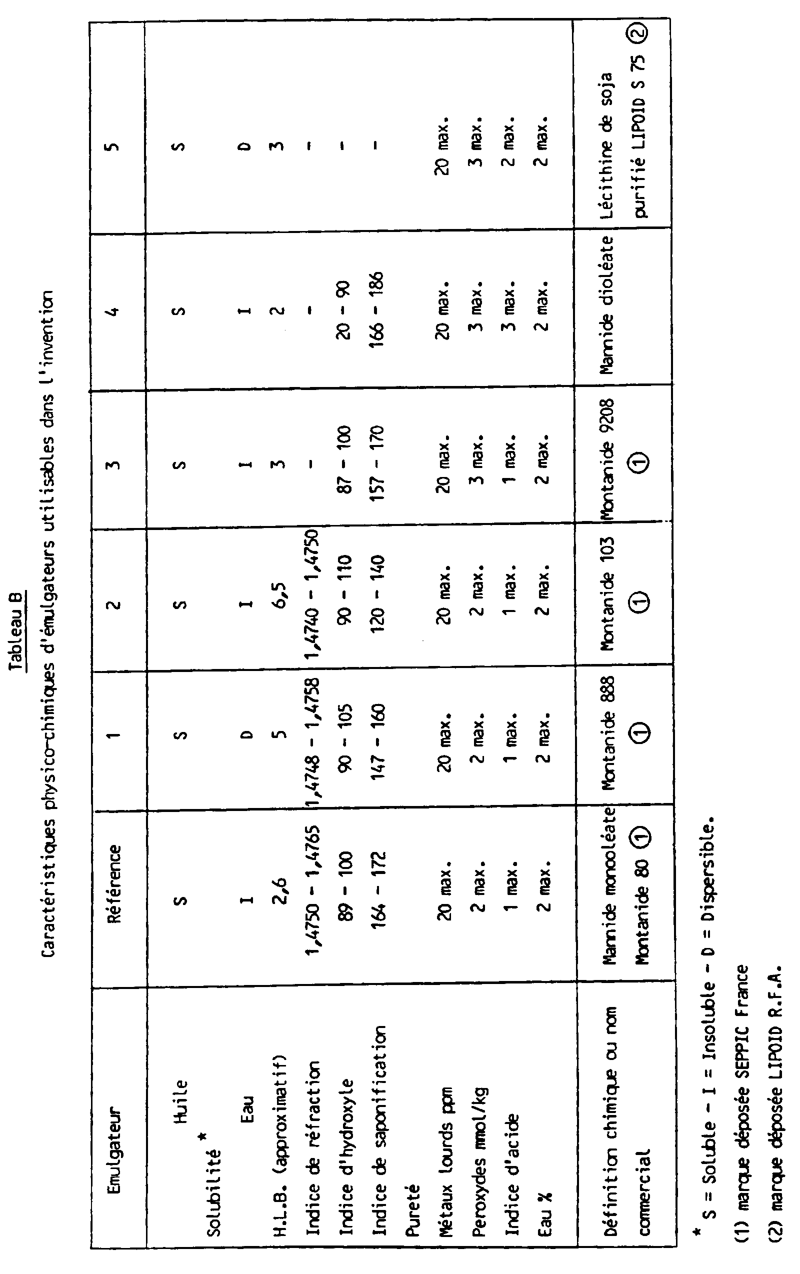

### Exemple 3

Cet exemple montre l'importance de la fluidité des vaccins sur leur tolérance par des animaux.

On réalise deux vaccins porcins contre la maladie d'AUJESZKY contenant le même milieu antigénique inactivé à la β-propiolactone. Les caractéristiques du vaccin A et du vaccin B (contenant un adjuvant type FIA sont mentionnées dans le tableau 3 - 1.

Le vaccin A se caractérise par une faible viscosité, une bonne injectabilité : il contient L'émulgateur 2 décrit dans le tableau (B).

Les deux vaccins contiennent la même huile minérale fluide.

L'expérimentation a eu lieu sur 2 lots de 6 porcs auxquels ont été administrés 2 fois, à 15 jours d'intervalle, 2 ml de vaccin par voie I.M. L'analyse sérologique etl'examen histologique ont eu lieu 59 jours après la première vaccination.

Les résultats sont consignés dans le tableau 3 - 2. On peut y remarquer que les taux d'anticorps sont voisins pour les 2 vaccins, mais que le vaccin A est beaucoup mieux toléré que le vaccin B, pour lequel 2 porcs ont présenté des comportements anormaux après chaque vaccination. Aux niveaux des sites d'injection, nécroses et suppurations - totalement inacceptables par les autorités sanitaires - sont pratiquement absentes du groupe A. Les granulomes macrophagiques et les infiltrats inflammatoires observés sont les témoins d'une réaction immunitaire intense.

**Tableau 3 - 1**

| Caractéristiques des vaccins porcins Aujeszky | | |
|---|---|---|
| | Vaccin A | Vaccin B |
| Caractéristiques de l'adjuvant | | |
| Composition | huile minérale 90 % émulgateur 2 : 10 % | huile minérale 89 % monooléate de mannide 11 % |
| Viscosité (mPa.s) | 35 | 40 |
| Indice de réfraction | 1,459 | 1,461 |

| Caractéristiques du vaccin | | |
|---|---|---|
| Composition % W/W | Adjuvant 70 | Adjuvant 50 |
| | Milieu 30 | Milieu 50 |
| Viscosité (BROOKFIELD M2V12) | 37 mPa.s | 1870 mPa.s |
| Sens | E/H | E/H |
| Conductivité | 0,2 µS | 0,4 µS |
| Stabilité 4°C | > 12 mois | > 12 mois |
| Aspect microscopique | gouttes 1 µ | gouttes 1-5 µ |

**Tableau 3 - 2**

| Résultats de la vaccination | | |
|---|---|---|
| | Vaccin A | Vaccin B |
| Sérologie | | |
| Titre en anticorps (log 2) (moyenne sur 5 animaux) | 2,5 | 2,0 |

| Histologie (fréquence sur 6 porcs) | | |
|---|---|---|
| Nécrose | 1 | 3 |
| Suppuration | 0 | 3 |
| Fibrose | 5 | 5 |
| Atrophie musculaire | 5 | 6 |
| Granulomes macrophagiques ou lymphocytaires | 5 | 4 |
| Infiltrat inflammatoire diffus | 2 | 2 |
| Réaction générale (modification du comportement) | 0 | 2 |

### Exemple 4

On réalise un vaccin antiaphteux pour bovins par mélange sous agitation mécanique d'une partie d'adjuvant contenant l'émulgateur 1 et d'une partie du milieu antigénique inactivé. L'adjuvant contient 5% d'une huile végétale.

Les caractéristiques physico-chimiques de l'adjuvant et du vaccin obtenu sont donnnées ci-dessous (tableau 4-1).

Le test d'efficacité immunologique donne les résultats résumés dans le tableau 4 - 2 qui peuvent être comparés à la préparation de l'exemple 1.

L'activité est maintenue sur des préparations stockées jusqu'à 2 ans.

La tolérance de la préparation injectée par voie intramusculaire chez le bovin est bonne. L'examen histologique révèle une inflammation légère des tissus au point d'injection avec présence de peu de nodules et de granulomes. La résorption de l'inflammation et la disparition microscopiques du produit injecté sont obtenues dans les 15 jours suivant l'injection.

**Tableau 4 - 1**

| Caractéristiques du vaccin antiaphteux | | |
|---|---|---|
| Caractéristiques de l'adjuvant | | |
| Composition | huile d'arachide | 5 % |
| | huile minérale fluide | 84 % |
| | émulgateur 1 | 11 % |
| Aspect | liquide huileux limpide, jaune clair, stable | |
| Viscosité | 40 mPa.s | |
| Indice d'acide | 0,1 | |
| Indice de réfraction | 1,460 environ | |
| Densité | 0,85 | |

| Caractéristiques du vaccin antiaphteux | | |
|---|---|---|
| Conductivité à 20°C | 1,5 microsiemens | |
| Viscosité à 20°C | 250 centistokes | |
| Stabilité à 4°C | très léger anneau d'huile | |
| Aspect microscopique | gouttelettes de taille inférieure à 1 µm | |
| Centrifugation | pas de séparation après 30 min à 3 000 tr/min. | |

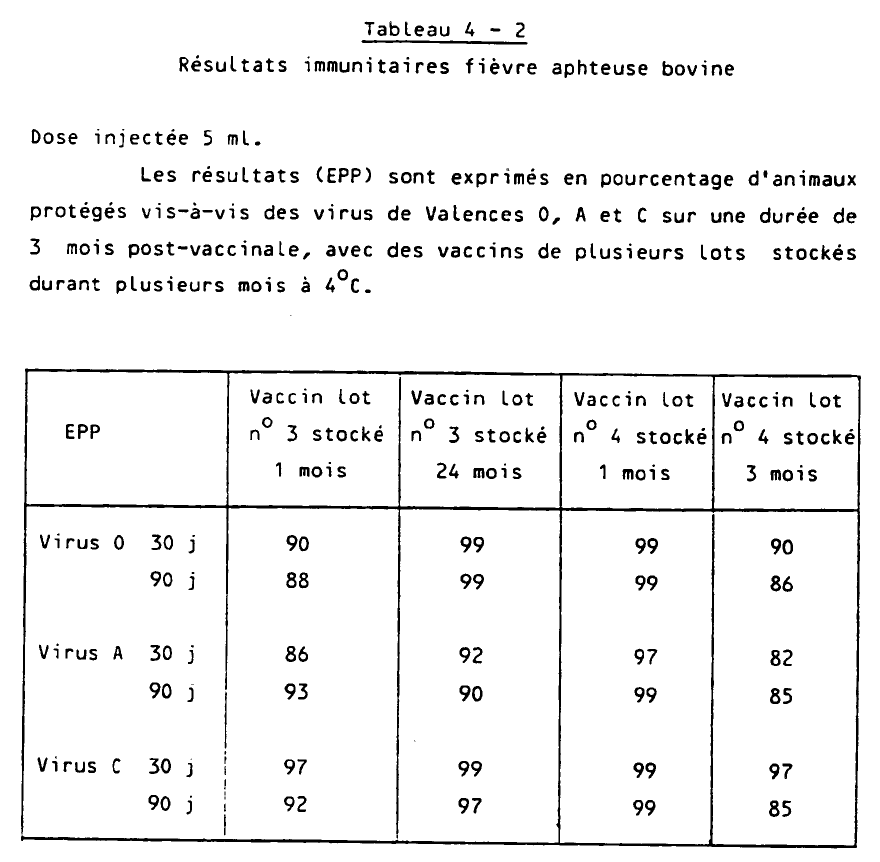

### Exemple 5

On vaccine des souris avec des vaccins constitués
- d'un milieu antigénique à 100 µg/ml d'albumine bovine
- d'un adjuvant huileux contenant
   soit une huile de synthèse (vaccin 5A),
   soit une huile de synthèse et une huile végétale (vaccin 5B).

Ces deux vaccins sont fluides et stables. Leurs caractéristiques physic-chimiques sont données dans le tableau 5-1.

Des lots de 10 souris SWISS sont vaccinés par 0,1 ml de ces vaccins. Un lot témoin reçoit le milieu antigénique non adjuvé.

Les taux d'anticorps, mesurés au cours du temps par une technique ELISA, sont donnés dans le tableau 5-2. On constate que les deux vaccins adjuvés se comportent de la même façon bien que le vaccin 5B contienne une huile métabolisable, et sont nettement plus efficaces que le vaccin non adjuvé. On remarquera l'apparition rapide des anticorps avec le vaccin contenant l'huile métabolisable.

**Tableau 5 - 1**

| Caractéristiques physico-chimiques du vaccin BSA | | |
|---|---|---|
| Vaccin | 5A | 5B |
| Caractéristiques de l'adjuvant | | |
| Composition | Polyisobutène 89% | Polyisobutène 44% |
| | émulgateur 2 11% | huile amande |
| | | douce à 45% |
| | | émulgateur 3 11% |
| Viscosité mPa.s | 45 | 50 |
| Aspect | liquide limpide clair | liquide limpide jaune |

| Caractéristiques du vaccin | | |
|---|---|---|
| Sens | E/H | E/H |
| Conductivité à 20°C | 0,9 µs | 1,2 µs |
| Viscosité mPa.s à 20°C | 100 | 130 |
| Stabilité | > 12 mois | > 12 mois |
| Aspect | émulsion homogène | émulsion homogène |

**Tableau 5 - 2**

| Taux d'anticorps chez la souris vaccinée par les vaccins BSA | | | | |
|---|---|---|---|---|
| Jours après vaccination | 14 | 28 | 56 | 125 |
| Vaccin non adjuvé | 60 | 157 | 102 | 105 |
| Vaccin 5A | 590 | 2420 | 3800 | 4560 |
| Vaccin 5B | 1400 | 2540 | 3760 | 4640 |

### Exemple 6

Une préparation injectable contenant des principes actifs hormonaux est réalisée par mélange d'une partie d'une suspension aqueuse etd'une partie d'un adjuvant huileux contenant des huiles métabolisables (squalène et huile d'amandes douces 84%) et une huile non métabolisable (squalane : 5 %) et l'émulgateur (3) 11 %.

Le vaccin obtenu a les caractéristiques suivantes :
- SENS: E/H
- CONDUCTIVITE: 0,14 µS
- VISCOSITE: 305 mPa.s
- STABILITE 4°C et 20°C: > 6 mois

Ces caractéristiques, en particulier viscosité et stabilité, ne peuvent être obtenues que grâce à la formulation spécifique de l'adjuvant huileux. En effet, un vaccin ayant le même rapport eau/huile mais formulé à partir de squalène et de monooléate de mannitol traditionnel est très visqueux (4400 mPa.s) d'injection extrêmement difficile et douloureuse, et peu stable (exsudation d'huile importante à 4 et 20°C).

La préparation selon l'invention a une efficacité suffisante et ne provoque chez le primate qu'une légère rougeur au point d'injection qui disparait au bout de quelques jours.

### Exemple 7

On réalise un vaccin Aujeszky par mélange de 30 parties d'un milieu viral inactivé titrant 2.10 9 DCP 50/ml et de 70 parties d'un adjuvant huileux contenant une huile végétale et l'émulgateur 3.

Le tableau 7 - 1 donne les caractéristiques de l'adjuvant et du vaccin obtenu. 2 ml de vaccin sont injectés à un lot de 5 porcs suivi d'un rappel à 15 jours. Une épreuve de résistance est pratiquée 15 jours après rappel. L'abattage et l'examen histologique ont eu lieu 5 semaines après le rappel.

Les résultats de l'expérimentation montrent dans tous les domaines une très bonne tolérance du vaccin. Ces résultats peuvent être comparés à ceux obtenus avec un vaccin huileux classique contenant un adjuvant type FIA et présentés à l'exemple 3 (vaccin B).

Il convient de noter tout particulièrement l'absence d'hyperthermie excessive, de nécroses et suppurations, la présence de granulomes macrophagiques discrets traduisant la réponse immunitaire et confirmant la bonne résistance à l'épreuve infectieuse.

**Tableau 7 - 1**

| Caractéristiques physico-chimiques du vaccin et de son adjuvant | | |
|---|---|---|
| Caractéristiques de l'adjuvant | | |
| Composition | Huile minérale fluide | 44 % |
| | Huile d'arachide raffinée | 44 % |
| | Emulgateur 3 | 12 % |
| Aspect | liquide huileux, jaune paille, limpide, stable, homogène. | |
| Viscosité à 20°C | 20 mPa.s | |
| Indice de réfraction | 1,466 | |
| Indice d'hydroxyle | 13 | |
| Indice de saponification | 105 | |

| Caractéristiques du vaccin | | |
|---|---|---|
| Sens | E/H | |
| Conductivité à 20°C | 1,9 µS | |
| Viscosité a 20°C | 38 mPa.s | |
| Aspect microscopique | homogène - gouttes de 1 µm | |
| Stabilité à 4°C | > 12 mois | |

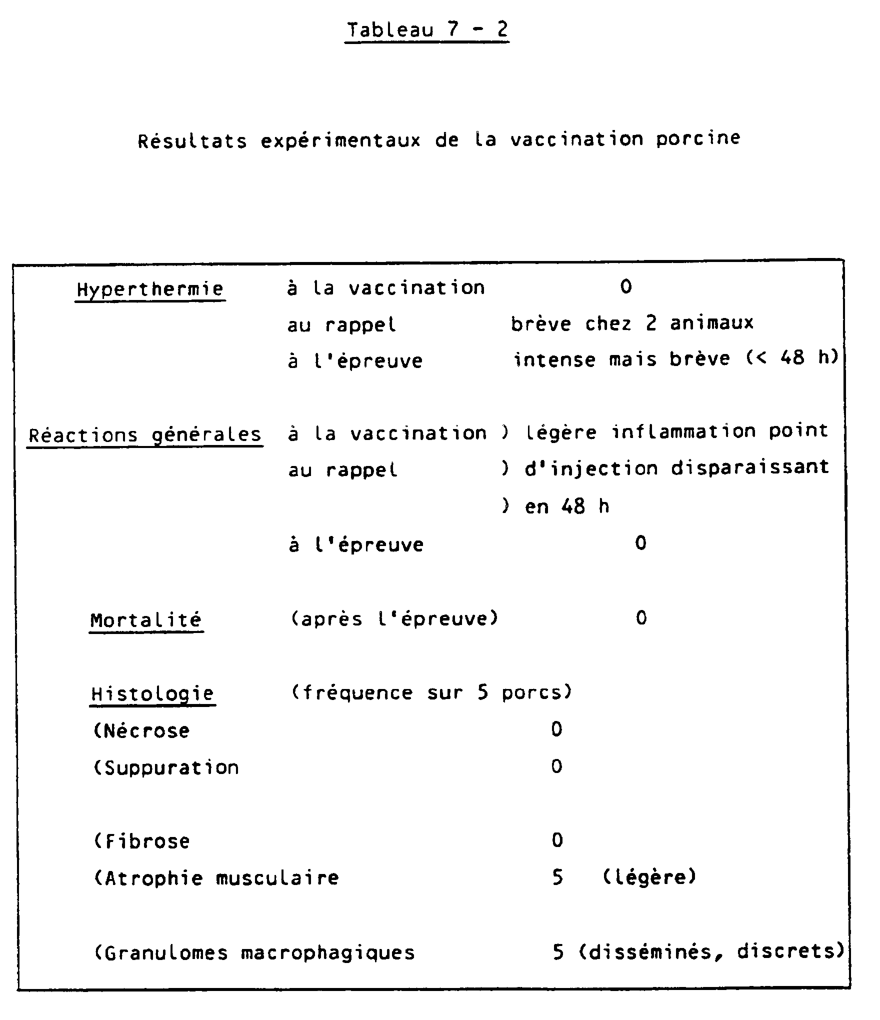

### Exemple 8

La même expérimentation que celle décrite à l'exemple 7 a été réalisée avec un vaccin contenant 70 parties d'un adjuvant composé de squalane d'un ester d'acide gras et d'un émulgateur légèrement hydrophile décrit sous référence 2 dans le tableau B et 30 parties de milieu antigénique Aujeszky. Le vaccin obtenu est très fluide et s'injecte facilement. Ses performances et ses caractéristiques sont données dans les tableaux 8 - 1 et 8 - 2.

On notera à nouveau l'absence de réactions d'intolérance importantes comparativement au vaccin classique de l'exemple 1B.

**Tableau 8 - 1**

| Caractéristiques physico-chimiques du vaccin porcin Aujeszky | | |
|---|---|---|
| Caractéristiques de l'adjuvant | | |
| Composition | Oléate d'éthyle | 6 % |
| | Squalane | 83 % |
| | Emulgateur 2 | 11 % |
| Aspect | liquide, limpide, transparent, jaune très clair | |
| (Viscosité à 20°C | 20 mPa.s | |
| (Indice de réfraction | 1,492 environ | |

| Caractéristiques du vaccin | | |
|---|---|---|
| Type | E/H | |
| Viscosité à 20°C | 88 mPa.s | |
| Conductivité à 20°C | 0,03 µS | |
| Stabilité à 4°C | légère exsudation d'huile | |

**Tableau 8 - 2**

| Résultats expérimentaux de la vaccination porcine | | |
|---|---|---|
| Hyperthermies | à la vaccination | très légères |
| | au rappel | > 41°C 24 h |
| | a l'épreuve | intenses mais brèves |
| Réactions générales | à la vaccination | 0 |
| | au rappel | légère inflammation au site d'injection |
| | à l'épreuve | peu prononcées |
| Mortalité (après l'épreuve) | | 0 |

| Histologie (fréquence sur 5 porcs) | | |
|---|---|---|
| (Abcès caséeux | | 2 |
| (Suppuration | | 0 |
| Fibrose | | 1 |
| Atrophie musculaire | | 5 |
| Granulomes macrophagiques | | 3 |

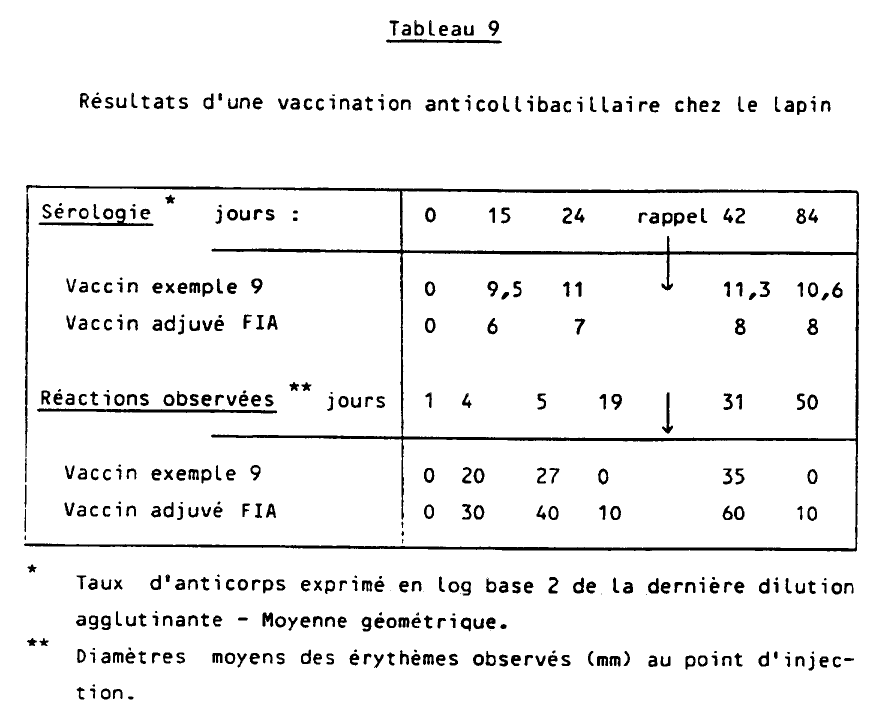

### Exemple 9

Un vaccin anticollibacillaire a été réalisé pour une vaccination chez le lapin.

Le milieu antigénique est une suspension de facteur d'attachement K 88 ab dans du sérum physiologique, à une concentration telle que la D 0 de cette suspension soit de 0,5 à 540 nm.

L'adjuvant utilisé est celui décrit à l'exemple 7 et au tableau 7 - 1.

Le vaccin contient 1/3 de milieu antigénique et 2/3 d'adjuvant. Ses caractéristiques sont les suivantes.
- SENS: E/H
- CONDUCTIVITE à 20°C: 2,0 µS
- VISCOSITE à 20°C: 40 mPa.s

Des lapins néozélandais de 6 semaines sont vaccinés par 1 ml de vaccin puis un rappel est effectué à 29 jours.

Les taux d'anticorps sont mesurés par une technique de micro-agglutination.

Le tableau 9 donne les résultats obtenus avec ce vaccin ainsi qu'avec un vaccin huileux classique formulé avec adjuvant de Freund incomplet (formule 50/50 ; viscosité > 3 000 mPa.s).

Les taux en anticorps avec le vaccin selon l'invention sont supérieurs ou égaux à ceux mesurés avec le vaccin classique. Les réactions au site d'injection sont très importantes avec le vaccin classique, surtout après le rappel.

Elles le sont beaucoup moins et régressent plus rapidement avec le vaccin de cet exemple.

### Exemple 10

Pour réaliser une préparation vaccinale contre la maladie de Newcastle chez les volailles, nous avons utilisé un adjuvant constitué de :

| | |
|---|---|
| Huile minérale fluide DRAKEOL® 6VR | 82 % |
| Huile de soja | 5 % |
| Emulgateur 2 | 13 % |

Cet adjuvant liquide, limpide présente les caractéristiques suivantes :
Indice acide inférieur à 0,5
Indice hydroxyle voisin de 11
Indice de réfraction de 1,459

Le vaccin est réalisé par mélange sous agitation mécanique de 7 parties d'adjuvant et de 3 parties de milieu allantoïdien contenant les antigènes Newcastle inactivés.

Les caractéristiques physico-chimiques du vaccin sont :
- Conductivité à 20°C: inférieure à 1 microsiemens
- Viscosité à 20°C: 45 centistokes
- Stabilité: pas de relargage d'huile, ni de déphasage du milieu à 4 C après 3 mois.

La préparation reste également stable à 37°C durant plus d'une semaine.

Des vaccins stockés 12 mois à 4°C et 1 mois à température ambiante, ainsi qu'un témoin constitué des antigènes viraux inactivés sans adjuvant et un vaccin à virus vivants sont injectés aux animaux.

L'étude est effectuée sur 3 séries de 10 poulets EOPS du type LEGHORN âgés de 4 semaines. L'injection se fait par voie sous-cutanée dans la région du cou par dose de 0,5 ml.

La mesure de l'efficacité immunologique est effectuée par titrage de l'inhibition de l'hémagglutination.

On prélève le sang de la volaille vaccinée dans la veine de l'aile au temps To, 4, 8, 12, 18 semaines et on mesure la teneur en hémagglutinine.

### - Titre d'inhibition de l'hématoagglutination

| | To | 4 sem. | 8 sem. | 12 sem. | 18 sem. |
|---|---|---|---|---|---|
| 1 (témoin) | 10 | 9 | 10 | 8 | 8 |
| 2 (vaccin selon l'invention) | 210 | 230 | 190 | 150 | 160 |
| 3 (vaccin virus vivants) | 305 | 210 | 120 | 70 | 50 |

On constate la supériorité de l'activité immunologique obtenue avec le vaccin de l'invention par rapport au témoin.

Contrairement à ce qui est observé avec le vaccin à virus vivants, l'activité persiste après plus de 4 mois, ce qui permet d'assurer la protection immunitaire des poulets vis-à-vis de la fièvre de Newcastle tout au long de leur durée de vie.

Sur le plan toxicité, on ne révèle aucune mortalité de la volaille vaccinée avec le vaccin selon l'invention, alors qu'un certain nombre de poulets (5 %) sont morts peu après injection du vaccin à virus vivants.

## Revendications

1. Emulsion injectable notamment utile comme vaccin ou vecteur de principe actif du type constituée de :
- 20 à 90 % en poids d'un adjuvant huileux liquide à 4°C et contenant un système émulgateur pharmaceutiquement acceptable, et
- 10 à 80 % en poids d'une phase hydrophile contenant les principes actifs ou antigènes, caractérisé en ce que ledit adjuvant huileux contient un mélange en toute proportion d'une ou plusieurs huiles métabolisables et d'une ou plusieurs huiles non métabolisables, et en ce que ledit système émulgateur est choisi de façon à former avec ledit mélange d'huiles une phase homogène et stable et à permettre l'obtention d'une émulsion stable présentant une viscosité inférieure à 800 mPa.S à 20°C.

2. Emulsion selon la revendication 1, dont l'huile métabolisable est d'origine végétale.

3. Emulsion selon la revendication 1, dont l'huile métabolisable est d'origine animale.

4. Emulsion selon la revendication 1, dont l'huile métabolisable est un ester résultant de la condensation d'un acide gras contenant de 12 à 24 atomes de carbone avec un alcool ou de la condensation d'un alcool gras contenant de 12 à 24 atomes de carbone avec un acide.

5. Emulsion selon la revendication 1, dont l'huile métabolisable est une huile de synthese de type alcane, alcène ou alcyne dont le nombre d'atomes de carbone moyen est d'au moins 16.

6. Emulsion selon la revendication 1, dont l'huile non métabolisable est d'origine minérale ou animale et possède un nombre moyen d'atomes de carbone au moins égal à 16.

7. Emulsion selon l'une des revendications 1 à 7, dont l'huile non métabolisable représente de 2 à 95%, de préférence 10 à 50% de l'adjuvant huileux.

8. Emulsion selon l'une des revendications 1 à 6, dont le ou les émulgateurs sont obtenus par condensation d'un acide gras Liquide à 20°C contenant au moins 16 atomes de carbone avec un polyol.

9. Adjuvant huileux liquide et stable à 4°C permettant d'obtenir une émulsion telle que définie selon l'une quelconque des revendications 1 à 8 caractérisé en ce qu'il est constitué d'un mélange d'une ou plusieurs huiles métabolisables et d'une ou plusieurs huiles non métabolisables et d'un, ou de plusieurs émulgateurs décrits dans la revendication 8.

## Claims

1. Injectable emulsion, particularly usable as a vaccine or active principle vehicle of the type consisting in:
- 20 to 90 % by weight of an oily adjuvant which is liquid at 4°C and containing a pharmaceutically acceptable emulsifier system, and
- 10 to 80 % by weight of a hydrophilic phase containing the active principles or antigens, characterized in that said oily adjuvant has a mixture, in any proportions, of one or more metabolizable oils and of one or more non-metabolizable oils, and in that said emulsifier system is selected in such a manner as to form a homogenous and stable phase with said mixture of oils and to make it possible to obtain a stable emulsion having a viscosity which is less than 800 mPa.S at 20°C.

2. Emulsion according to claim 1, in which the metabolizable oil is of vegetable origin.

3. Emulsion according to claim 1, in which the metabolizable oil is of animal origin.

4. Emulsion according to claim 1, in which the metabolizable oil is an ester resulting from the condensation of a fatty acid containing from 12 to 24 carbon atoms with an alcohol, or from the condensation of a fatty alcohol containing from 12 to 14 carbon atoms with an acid.

5. Emulsion according to claim 1, in which the metabolizable oil is a synthetic oil of the alkane, alkene or alkyne type in which the mean number of carbon atoms is at least 16.

6. Emulsion according to claim 1, in which the non-metabolizable oil is of mineral or animal origin and has a mean number of carbon atoms equal to at least 16.

7. Emulsion according to one of claims 1 to 7, in which the non-metabolizable oil represents from 2 to 95 %, preferably 10 to 50 % of the oily adjuvant.

8. Emulsion according to one of claims 1 to 6, in which the emulsifier or emulsifiers are obtained by condensing a fatty acid which is liquid at 20°C and contains at least 16 carbon atoms with a polyol.

9. Oily adjuvant which is liquid and stable at 4°C which enables obtaining an emulsion such as defined according to any of claims 1 to 8, characterised in that it consists of a mixture of one or more metabolizable oils, one or more non-metabolizable oils, and one or more emulsifiers described in claim 8.

## Patentansprüche

1. Injizierbare Emulsion, die insbesondere als Impfstoff oder Wirkstoffträger brauchbar ist, der Art, die aus:
- 20 bis 90 Gew.% eines ölförmigen, bei 4 °C flüssigen und ein pharmazeutisch annehmbares Emulgiersystem enthaltenden Stoffes und
- 10 bis 80 Gew.% einer hydrophilen Phase besteht, die die Wirkstoffe oder Antigene enthält,
**dadurch gekennzeichnet**, daß der ölförmige Stoff eine Mischung in jedem Verhältnis eines oder mehrerer metabolisierbarer Öle und eines oder mehrer nicht metabolisierbarer Öle enthält und daß das Emulgiersystem derart gewählt wird, um mit der Mischung von Ölen eine homogene und stabile Phase zu bilden und um das Erhalten einer stabilen Emulsion zu ermöglichen, die eine Viskosität unter 800 mPa · s bei 20 °C aufweist.

2. Emulsion nach dem Anspruch 1, deren metabolisierbares Öl pflanzlichen Ursprungs ist.

3. Emulsion nach dem Anspruch 1, deren metabolisierbares Öl tierischen Ursprungs ist.

4. Emulsion nach dem Anspruch 1, deren metabolisierbares Öl ein Ester ist, der sich aus der Kondensation einer 12 bis 24 Kohlenstoffatome enthaltenden Fettsäure mit einem Alkohol oder aus der Kondensation eines 12 bis 24 Kohlenstoffatome enthaltenden Fettalkohols ergibt.

5. Emulsion nach dem Anspruch 1, deren metabolosierbares Öl ein Syntheseöl vom Alkan-, Alken- oder Alkintyp ist, dessen mittlere Kohlenstoffatomzahl wenigstens 16 ist.

6. Emulsion nach dem Anspruch 1, deren nicht metabolisierbares Öl mineralischen oder tierischen Ursprungs ist und eine mittlere Kohlenstoffatomzahl von Wenigstens gleich 16 besitzt.

7. Emulsion nach einem der Ansprüche 1 bis 6, deren nicht metabolisierbares Öl 2 bis 95 %, vorzugsweise 10 bis 50 % des ölförmigen Stoffes darstellt.

8. Emulsion nach einem der Ansprüche 1 bis 6, deren Emulgator oder Emulgatoren durch Kondensation einer bei 20 °C flüssigen Fettsäure, die wenigstens 16 Kohlenstoffatome enthält, mit einem Polyol erhalten werden.

9. Ölförmiger und bei 4 °C stabiler Stoff, der es erlaubt, eine Emulsion nach einem der Ansprüche 1 bis 8 herzustellen, dadurch gekennzeichnet, daß er aus einem Gemisch aus einem oder mehreren metaboliosierbaren Ölen, einem oder mehreren nicht metabolisierbaren Ölen und einem oder mehreren im Anspruch 5 beschriebenen Emulgatoren besteht.
